# EUROPÄISCHE PATENTSCHRIFT

(11) **EP 0 858 995 B1**
(45) Veröffentlichungstag und Bekanntmachung des Hinweises auf die Patenterteilung: **12.12.2001**
(21) Anmeldenummer: 98101694.2
(22) Anmeldetag: 02.02.1998
(51) Int. Cl.: C07D 209/86

(54) **Verfahren zur Herstellung von N-Alkylcarbazolen**
Process for the preparation of N-alkyl carbazoles
Procédé pour la préparation de N-alkyl-carbazoles

(30) Priorität: 13.02.1997 DE 19705466
(43) Veröffentlichungstag der Anmeldung: 19.08.1998
(73) Patentinhaber: Clariant GmbH, 65929 Frankfurt am Main (DE)
(72) Erfinder: Bauer, Wolfgang, Dr., 63477 Maintal (DE); Delpy, Klaus, Dr., 63128 Dietzenbach (DE); Nagl, Gert, Dr., 61338 Niederdorfelden (DE); Unverdorben, Leonhard, Dr., 61130 Nidderau (DE)

(56) Entgegenhaltungen:
- EP-A- 0 557 993
- CH-A- 342 961
- DE-C- 4 324 707
- H. NISHI ET AL.: BULL. CHEM. SOC. JPN, Bd. 54, 1981, Seiten 1897-8, XP002065104

## Beschreibung

Die vorliegende Erfindung betrifft ein Verfahren zur Herstellung von N-Alkyl-carbazolen durch Umsetzung von Carbazolen mit Alkylhalogeniden in Gegenwart eines Katalysators.

N-Alkylcarbazole, insbesondere N-Ethylcarbazol, sind wichtige Zwischenprodukte zur Herstellung wertvoller Farbstoffe und Pigmente (siehe beispielsweise Ullmann's Enzyklopädie der Technischen Chemie, 3. Auflage, Band 5, Seite 80 und 4.Auflage, Band 9, Seite 120; W. Herbst u. K. Hunger, "Industrielle Pigmente ", VCH Weinheim, S. 521 - 527, 1987).
Die Einführung einer N-Alkylgruppe in Carbazol erfolgt beispielsweise durch Alkylierung von Carbazol bzw. Carbazoliden mit Alkylhalogeniden, Dialkylsulfaten sowie Arensulfonsäure-alkylestern ( siehe beispielsweise Houben-Weyl, Methoden der Organischen Chemie, Band E 6a (1994), S.975-976).
Weiterhin können als Alkylierungsmittel Diethyl-N-(o-tolyl)-phosphoramidate (J.Heterocycl.Chem. 18, 315 (1981), Dialkoxycarbenium-tetrafluoroborate (Liebigs Ann. Chem. 1987, 509), Diethylcarbonat (DE-A 4324707) sowie Oxalsäurediethylester (Tetrahedron 46, 6113 (1990)) eingesetzt werden.
Die Verwendung von Dialkylsulfaten oder Arensulfonsäurealkylestern ist nachteilig, weil wegen der toxischen und cancerogenen Eigenschaften dieser Verbindungen aus ökologischen und arbeitshygienischen Gründen aufwendige Maßnahmen getroffen werden müssen.
Bei Einsatz von Diethyl-N-(o-tolyl)-phosphoramidaten bzw. Dialkoxycarbeniumtetrafluoroboraten werden lediglich Ausbeuten von 30% bzw. 67% der theoretischen Ausbeute an N-Ethylcarbazol erzielt. Durch den Anfall und die Entsorgung der somit entstehenden Nebenprodukte ergeben sich erhebliche wirtschaftliche und umweltrelevante Nachteile.
Bei Verwendung von Diethylcarbonat als Alkylierungsmittel in Gegenwart basischer Katalysatoren werden sehr hohe Temperaturen von 170°C bis 240°C und lange Reaktionszeiten von bis zu 24 Stunden benötigt, um Ausbeuten >90% der theoretischen Ausbeute an N-Ethylcarbazol zu erzielen. Dieser Prozeß weist somit einen unwirtschaftlich hohen Energiebedarf und eine ungünstige Raum-/ Zeitausbeute auf.
N-Ethylcarbazol wird technisch durch Umsetzung von Carbazol mit Kaliumhydroxid und Ethylierung des intermediär gebildeten Carbazol-Kaliums mit Ethylchlorid hergestellt (siehe beispielsweise BIOS Final Report 968, S.197). Auch bei diesem Verfahren führen lange Reaktionszeiten und hohe Reaktionstemperaturen zu ökonomischen Nachteilen.
Um diese Nachteile zu beheben, sind bei Alkylierungsreaktionen von Carbazol bzw. Alkali-Carbazoliden eine Reihe von Katalysatoren eingesetzt worden.
Allerdings werden bei der Ethylierung von Carbazol mit Ethylbromid in einem Zweiphasengemisch aus Benzol und 50%iger wäßriger Natronlauge in Gegenwart von Benzyl-triethyl-ammoniumchlorid als Phasentransferkatalysator lediglich 86,2% der theoretischen Ausbeute an N-Ethylcarbazol erzielt (Bull.Chem.Soc.Jpn. 54, 1897 (1981)).
Werden als Phasentransferkatalysatoren bei der Reaktion von Carbazol mit Ethylbromid im Zweiphasengemisch Benzol/Kaliumhydroxid Polyethylenglykoldialkylether verwendet, müssen diese Katalysatoren in äquimolaren Mengen eingesetzt werden, um Ausbeuten von 92% der Theorie an N-Ethylcarbazol zu erreichen (Bull.Chem.Soc.Jpn. 56, 280(1983)).
In diesen Fällen ist der Einsatz des besonders reaktiven Ethylbromids als Alkylierungsmittel bzw. eine äquimolare Menge an Phasentransferkatalysator erforderlich, wodurch auch diese Prozesse unwirtschaftlich und ökologisch nachteilig sind.
In der EP-A 557993 wird ein Verfahren zu Herstellung von N-Alkylcarbazolen beschrieben, welches dadurch gekennzeichnet ist, daß man Carbazol mit Ethylchlorid in einem Zweiphasensystem aus beispielsweise 1,2-Dichlorbenzol und 48%iger wäßriger Natronlauge unter Verwendung von Trialkylaminen als Katalysatoren umsetzt. Dabei werden bei Reaktionszeiten von 9 Stunden und
Reaktionstemperaturen von 100°C Ausbeuten weit über 90% der theoretischen Ausbeute an N-Ethylcarbazol erzielt. Technisch nachteilig an diesem Verfahren ist allerdings, daß der eingesetzte Katalysator aus der zunächst bei der Alkylierung erhaltenen organischen Produktphase durch einen zusätzlichen Waschprozess mit wäßriger Schwefelsäure entfernt werden muß.

Überraschenderweise wurde nun gefunden, daß durch den Einsatz bestimmter Katalysatoren die oben geschilderten Nachteile, insbesondere des Verfahrens gemäß EP-A 557993, vermieden werden können.

Gegenstand der vorliegenden Erfindung ist ein Verfahren zur Herstellung von N-Alkyl-carbazolen der allgemeinen Formel I 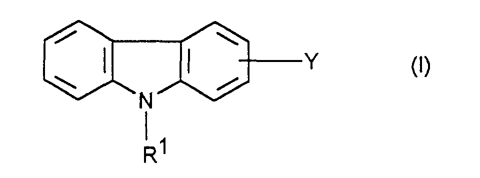 worin
- R¹: (C₁-C₆)-Alkyl und
- Y: Wasserstoff, (C₁-C₆)-Alkyl, (C₁-C₆)-Alkoxy, Nitro oder Halogen bedeuten,
durch Umsetzung eines Carbazols der allgemeinen Formel II 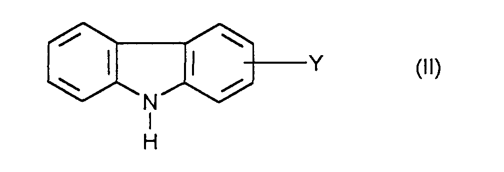 mit einem Alkylhalogenid der allgemeinen Formel III

R¹-X III

worin X für ein Halogenatom steht,
in einem inerten Lösungsmittel in Gegenwart einer anorganischen Base und eines Katalysators der allgemeinen Formel IV

R²R³N-(CH₂)ₙ-NR⁴R⁵ IV

worin
- n: für eine ganze Zahl von 2 bis 8,
- R², R³ und R⁴: unabhängig voneinander Wasserstoff oder (C₁-C₄)-Alkyl und
- R⁵: Wasserstoff, (C₁-C₄)-Alkyl, Amino-(C₁-C₄)-alkyl oder N,N-Di- (C₁-C₄)-alkylamino-(C₁-C₄)-alkyl
bedeuten.

Alkylgruppen können geradkettig oder verzweigt sein. Für R¹ stehendes (C₁-C₆)-Alkyl bedeutet beispielsweise Methyl, Ethyl, Propyl, n-Butyl, i-Butyl, sek.-Butyl, tert.-Butyl, Pentyl oder Hexyl. Analoges gilt für Y stehendes Alkoxy, sowie für R², R³, R⁴ oder R⁵ stehendes (C₁-C₄)-Alkyl, Amino-(C₁-C₄)-alkyl oder N,N-Di- (C₁-C₄)-alkylamino-(C₁-C₄)-alkyl.
Ein für Y oder X stehendes Halogenatom kann beispielsweise Fluor, Chlor, Brom oder lod sein.

In bevorzugten Verbindungen der allgemeinen Formel I stehen R¹ für (C₁-C₄)-Alkyl, besonders bevorzugt für Methyl oder Ethyl und Y für Wasserstoff.

Alkylhalogenide der allgemeinen Formel III sind bevorzugt Alkyliodide, -bromide oder -chloride. Besonders bevorzugt sind Alkylchloride. Beispielsweise können Methylchlorid, Methylbromid, Methyliodid, Ethylchlorid, Ethylbromid, n-Propylchlorid, n-Propylbromid, iso-Butylchlorid, iso-Butylbromid, n-Pentylchlorid oder n-Hexylchlorid eingesetzt werden. Ganz besonders Bevorzugt ist Ethylchlorid.
Die Alkylhalogenide der allgemeinen Formel III werden bevorzugt in Mengen von 1,0 bis 1,8 Mol, besonders bevorzugt 1,1 bis 1,5 Mol pro Mol Carbazol der allgemeinen Formel I eingesetzt.

In dem erfindungsgemäßen Verfahren können als anorganische Basen insbesondere Alkalialkoholate und Hydroxide, Oxide, Carbonate sowie tert. Phosphate der Alkalimetalle und Erdalkalimetalle oder deren Gemische eingesetzt werden. Beispiele sind Natriummethylat, Natriumethylat, Kalium-tert.butylat, Lithiumhydroxid, Natriumhydroxid, Kalium-hydroxid, Natriumcarbonat, Kaliumcarbonat, Trinatriumphosphat, Calciumoxid, Calciumhydroxid und Bariumhydroxid.
Bevorzugte anorganische Basen sind Lithiumhydroxid, Natriumhydroxid und Kaliumhydroxid, besonders bevorzugt sind Natriumhydroxid und Kaliumhydroxid. Alkalimetallhydroxide werden ganz besonders bevorzugt in Form konzentrierter, insbesondere 40 bis 60%iger, wäßriger Lösungen eingesetzt.
Die anorganischen Basen werden bevorzugt in Mengen von 0,9 bis 3,0 Val, besonders bevorzugt 1,1 bis 2,0 Val pro Mol Carbazol der allgemeinen Formel I eingesetzt.

Als inerte Lösungsmittel können in dem erfindungsgemäßen Verfahren die üblicherweise in der organischen Chemie verwendeten und dem Fachmann bekannten inerten Lösungsmittel eingesetzt werden. Beispiele sind insbesondere: Toluol, o-Xylol, m-Xylol, p-Xylol, Mesitylen, 2-Chlortoluol, 3-Chlortoluol, 4-Chlortoluol, Monochlorbenzol, 1,2-Dichlorbenzol, 1,3-Dichlorbenzol, 1,4-Dichlorbenzol, N-Ethylcarbazol sowie deren Gemische.
Bevorzugte Lösungsmittel sind Toluol, Xylol, Monochlorbenzol und 1,2-Dichlorbenzol , besonders bevorzugt ist 1,2 - Dichlorbenzol.
Die inerten Lösungsmittel werden bevorzugt in Mengen von 0,5 bis 10 Gewichtsteilen, besonders bevorzugt von 1 bis 5 Gewichtsteilen pro Gewichtsteil Carbazol der allgemeinen Formel II eingesetzt.

In den Katalysatoren der allgemeinen Formel IV bedeutet n bevorzugt eine ganze Zahl von 2 bis 6, besonders bevorzugt die Zahl 3 und R², R³, R⁴ und R⁵ stehen bevorzugt für Wasserstoff, Methyl oder Ethyl, besonders bevorzugt für Methyl. Beispielsweise können Ethylendiamin, 2-Ethylamino-ethylamin, 2-Diethylaminoethylamin, 3-Dimethylaminopropylamin, 3-Diethylamino-propylamin, N,N,N',N'-Tetramethyl-1,3-propandiamin, N,N-Diethyl-N',N'-dimethyl-1,3-propandiamin, N,N,N',N'-Tetramethyl-1,4-butandiamin, 1-Diethylamino-4-aminopentan, N,N,N',N'-Tetramethyl-1,6-hexandiamin, N,N-Dimethyldipropylentriamin, Bis-(3-dimethylaminopropyl)-amin, N,N,N',N",N"-Pentamethyldiethylentriamin und N,N,N',N",N"-Pentamethyldipropylen-triamin eingesetzt werden.

Besonders bevorzugt sind N,N-N',N'-Tetramethyl-1,3-propandiamin und N,N-Diethyl-N',N'-dimethyl-1,3-propandiamin und ganz besonders bevorzugt ist N,N,N',N'-Tetramethyl-1,3-propandiamin.

Die Katalysatoren der allgemeinen Formel IV werden bevorzugt in Mengen von 0,001 bis 0,05 Mol, besonders bevorzugt 0,005 bis 0,02 Mol pro Mol eingesetztes Carbazol, eingesetzt.
Im Vergleich zum nächstvergleichbaren Stand der Technik sind dies deutlich geringere Mengen zur Erzielung eines gleich hohen Umsetzungsgrades. Daraus ergeben sich ökologische und wirtschaftliche Vorteile. Durch den Einsatz der Katalysatoren der allgemeinen Formel IV kann weiterhin überraschenderweise auf eine Nachbehandlung der Produktphase mit wäßriger Schwefelsäure verzichtet werden, da sich der Katalysator nach der Phasentrennung in der wäßrigen Phase und nicht bzw. nur in Spuren in der organischen Produktphase befindet. Durch diese vereinfachte Aufarbeitung ist ein weiterer wirtschaftlicher Vorteil des erfindungsgemäßen Verfahrens im Vergleich zum Stand der Technik gegeben.

Das erfindungsgemäße Verfahren wird bevorzugt bei Temperaturen von 70 bis 160°C, besonders bevorzugt 80 bis 130°C, durchgeführt.

Das erfindungsgemäße Verfahren kann beispielsweise in der Weise durchgeführt werden, daß man ein Carbazol der allgemeinen Formel II, inertes Lösungsmittel, wäßriges Alkalihydroxid und Katalysator der allgemeinen Formel IV vorlegt und in einem Autoklaven bei einer Reaktionstemperatur von 100°C und bei einem Druck von max. 5 bar unter Rühren Ethylchlorid zudosiert. Anschließend wird 2 bis 10 Stunden bei 100°C nachgerührt bis der Gehalt an Carbazol, bezogen auf gebildetes N-Ethylcarbazol unter einem gewünschten Grenzwert, beispielsweise <1% oder <0,1% liegt.
Nach beendeter Reaktion werden ausgefallene anorganische Salze mit Wasser gelöst. Die nach der Phasentrennung erhaltene organische Phase kann aufgrund der hohen Ausbeute von >95% der theoretischen Ausbeute an N-Ethylcarbazol und des hohen Reinheitsgrads ohne weitere Reinigungsoperation direkt für Folgereaktionen, beispielsweise Nitrierungen, verwendet werden.

Die folgenden Beispiele dienen zur Erläuterung der Erfindung, ohne sie einzuschränken. Bei den Gehaltsangaben handelt es sich um Gewichtsprozente.

### Beispiel 1

In einen beheizbaren Hastelloy-Autoklaven mit 3 Liter Fassungsvermögen werden 502 g Carbazol (3,0 Mol), 680 g o-Dichlorbenzol, 480 g 50 %-ige Natronlauge (6,0 Mol) und 4,4 g N,N,N',N'-Tetramethyl-1,3-propandiamin (0,034 Mol) vorgelegt. Der Autoklav wird mit N₂ inertisiert, verschlossen und auf 100°C Innentemperatur aufgeheizt. In einen Vorlage-Autoklaven, der über eine absperrbare Kapillare mit dem Reaktionsautoklaven verbunden ist, werden 273 g Ethylchlorid (4,2 Mol) auf 75 bis 80°C Innentemperatur erhitzt, so daß sich ein Druck von bis max. 5 bar (alle Druckangaben Überdruck) aufbaut. In den Reaktionsautoklaven dosiert man nun bei einer Innentemperatur von 100°C das Ethylchlorid aufgrund der Druckdifferenz über ein Nadelventil so zu, daß der Innendruck im Reaktionsautoklaven von 4 bar nicht überschritten wird. Die Gesamtdosierzeit beträgt 2 Stunden. Nach dem Zudosieren läßt man 4 Stunden bei 100°C nachrühren, danach unter Rühren auf ca. 40°C abkühlen und füllt dann das Reaktionsgemisch aus dem Autoklaven in einen Stutzen. Reste des Reaktionsgemisches werden aus dem Autoklaven nacheinander mit 400 g o-Dichlorbenzol und 1000 g Wasser in den Stutzen gespült. Der Stutzeninhalt wird solange kräftig gerührt, bis das Natriumchlorid sich in der wässrigen Phase vollständig aufgelöst hat und anschließend in einen Scheidetrichter überführt. Nach Abtrennung der wässrigen Phase werden 1670 g einer Lösung von N-Ethylcarbazol in o-Dichlorbenzol erhalten. Nach gaschromatographischer Analyse enthält diese Lösung 34,8% N-Ethylcarbazol, <0,1% Carbazol und <0,01% N,N,N',N'-Tetramethyl-1,3-propandiamin. Die Umsetzung ist somit vollständig und die Ausbeute beträgt 99,3 % der Theorie.

### Vergleichsbeispiel 1

In der in Beispiel 1 beschriebenen Apparatur werden 502 g Carbazol (3,0 Mol), 680 g o-Dichlorbenzol, 480 g 50%-ige Natronlauge (6,0 Mol) und 3,4 g Triethylamin (0,034 Mol) vorgelegt. Analog der Vorgehensweise in Beispiel 1 werden 273 g Ethylchlorid (4,2 Mol) zudosiert. Die Gesamtdosierzeit beträgt 6 Stunden. Nach dem Zudosieren läßt man 4 Stunden bei 100°C nachrühren, danach unter Rühren auf ca. 40°C abkühlen und füllt das Reaktionsgemisch aus dem Autoklaven in einen Stutzen. Reste des Reaktionsgemisches werden aus dem Autoklaven nacheinander mit 400 g o-Dichlorbenzol und 1000 g Wasser in den Stutzen gespült. Der Stutzeninhalt wird solange kräftig gerührt, bis das Natriumchlorid sich in der wässrigen Phase vollständig aufgelöst hat und anschließend filtriert. Der Filterkuchen wird mit 100 g o-Dichlorbenzol gewaschen und trocken gesaugt. Das Filtrat wird in einen Scheidetrichter überführt. Nach Abtrennung der wässrigen Phase werden 1736 g einer Lösung von N-Ethylcarbazol in o-Dichlorbenzol erhalten. Nach gaschromatographischer Analyse enthält diese Lösung 31,1% N-Ethyl-carbazol, 0,6% Carbazol und 0,08% Triethylamin. Der Filterkuchen wiegt nach dem Trocknen 28 g und ist nach Dünnschichtchromatographie reines Carbazol. Die Umsetzung des Carbazols beträgt somit nur 92,3 %.

### Beispiel 2

In der in Beispiel 1 beschriebenen Apparatur werden 502 g Carbazol (3,0 Mol), 680 g o-Dichlorbenzol, 480 g 50%-ige Natronlauge (6,0 Mol) und 20,6 g N,N,N',N'-Tetramethyl-1,6-hexandiamin (0,12 Mol) vorgelegt und 273 g Ethylchlorid (4,2 Mol) in 2 Stunden zudosiert. Man läßt 3 Stunden bei 100°C nachrühren, kühlt danach unter Rühren auf ca. 40°C ab und füllt das Reaktionsgemisch aus dem Autoklaven in einen Stutzen. Reste des Reaktionsgemisches werden aus dem Autoklaven nacheinander mit 400 g o-Dichlorbenzol und 1000 g Wasser in den Stutzen gespült. Der Stutzeninhalt wird solange kräftig gerührt, bis das Natriumchlorid sich in der wässrigen Phase vollständig aufgelöst hat, und anschließend in einen Scheidetrichter überführt. Nach Abtrennung der wässrigen Phase werden 1680 g einer Lösung von N-Ethylcarbazol in o-Dichlorbenzol erhalten. Nach gaschromatographischer Analyse enthält diese Lösung 34,7% N-Ethylcarbazol und <0,1% Carbazol. Die Umsetzung ist somit vollständig und die Ausbeute beträgt 99,6 % der Theorie.

### Beispiel 3

N-Ethylcarbazol wird hergestellt wie in Beispiel 2 angegeben mit dem einzigen Unterschied, daß anstelle von 20,6 g (0,12 Mol) N,N,N',N'-Tetramethyl-1,6-hexandiamin 13,9 g (0,12 Mol) N,N,N',N'-Tetramethyl-1,2-ethandiamin eingesetzt werden. Man erhält 1675 g einer Lösung von N-Ethylcarbazol in o-Dichlorbenzol. Nach gaschromatographischer Analyse enthält diese Lösung 34,5 % N-Ethylcarbazol und < 0,1 % Carbazol. Die Umsetzung ist somit vollständig und die Ausbeute beträgt 98,8 % der Theorie.

In der folgenden Tabelle sind weitere Beispiele für N-Alkylcarbazole der allgemeinen Formel I aufgeführt, welche nach dem erfindungsgemäßen Verfahren unter Verwendung der in Spalte 3 aufgeführten Alkylierungsmittel der allgemeinen Formel III und der in Spalte 4 aufgeführten Katalysatoren der allgemeinen Formel IV mit hohen Ausbeuten hergestellt werden können.

| Beispiel | Carbazol der allgemeinen Formel II | Alkylhalogenid der allgemeinen. Formel III | Katalysator der allgemeinen Formel IV |
|---|---|---|---|
| 4 | Carbazol | Methylbromid | N,N,N',N'-Tetramethyl-1,3-propandiamin |
| 5 | Carbazol | Hexylchlorid | N,N,N',N'-Tetramethyl-1,3-propandiamin |
| 6 | Carbazol | Ethylchlorid | N,N-Diethyl-1,3-propandiamin |
| 7 | 2-Methylcarbazol | Ethylchlorid | N,N,N',N'-Tetramethyl-1,3-propandiamin |
| 8 | 1-Methoxycarbazol | Ethylchlorid | N,N,N',N'-Tetramethyl-1,3-propandiamin |
| 9 | 3-Chlorcarbazol | Ethylchlorid | N,N,N',N'-Tetramethyl-1,3-propandiamin |
| 10 | 3-Nitrocarbazol | Ethylchlorid | N,N,N',N'-Tetramethyl-1,3-propandiamin |

## Patentansprüche

1. Verfahren zur Herstellung von N- Alkyl-carbazolen der allgemeinen Formel I 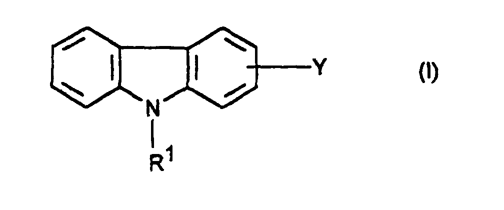 worin
R¹ (C₁-C₆)-Alkyl und
Y Wasserstoff, (C₁-C₆)-Alkyl, (C₁-C₆)-Alkoxy, Nitro oder Halogen bedeuten,
durch Umsetzung eines Carbazols der allgemeinen Formel II 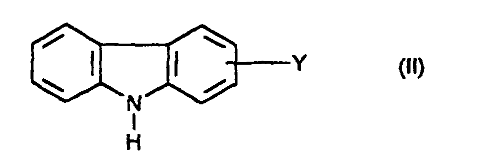 mit einem Alkylhalogenid der allgemeinen Formel III
R¹-X III
worin X für ein Halogenatom steht,
in einem inerten Lösungsmittel in Gegenwart einer anorganischen Base und eines Katalysators der allgemeinen Formel IV
R²R³N-(CH₂)n-NR⁴R⁵ IV
worin
n für eine ganze Zahl von 2 bis 8,
R², R³ und R⁴ unabhängig voneinander Wasserstoff oder (C₁-C₄)-Alkyl und
R⁵ Wasserstoff, (C₁-C₄)-Alkyl,
bedeuten.

2. Verfahren nach Anspruch 1, **dadurch gekennzeichnet, daß** in der Verbindung der allgemeinen Formel I R¹ Methyl oder Ethyl und Y Wasserstoff bedeuten.

3. Verfahren nach Anspruch 1 und/oder 2, **dadurch gekennzeichnet, daß** als Alkylhalogenide der allgemeinen Formel III Methylchlorid, Methylbromid, Methyliodid, Ethylchlorid, Ethylbromid, n-Propylchlorid, n-Propylbromid, iso-Butylchlorid, iso-Butylbromid, n-Pentylchlorid oder n-Hexylchlorid eingesetzt werden.

4. Verfahren nach einem oder mehreren der Ansprüche 1 bis 3, **dadurch gekennzeichnet, daß** die Alkylhalogenide der allgemeinen Formel III in Mengen von 1,0 bis 1,8 Mol, pro Mol Carbazol der allgemeinen Formel II eingesetzt werden.

5. Verfahren nach Anspruch 4, **dadurch gekennzeichnet, daß** Alkylhalogenide der allgemeinen Formel III in Mengen von 1,1 bis 1,5 Mol pro Mol Carbazol der allgemeinen Formel II eingesetzt werden.

6. Verfahren nach einem oder mehreren der Ansprüche 1 bis 5, **dadurch gekennzeichnet, daß** als anorganische Basen Alkalialkoholate und Hydroxide, Oxide, Carbonate sowie tert. Phosphate der Alkalimetalle und Erdalkalimetalle oder deren Gemische einsetzt werden.

7. Verfahren nach einem oder mehreren der Ansprüche 1 bis 6, **dadurch gekennzeichnet, daß** die anorganischen Basen in Mengen von 0,9 bis 3,0 Val, pro Mol Carbazol der allgemeinen Formel II eingesetzt werden.

8. Verfahren nach Anspruch 7, **dadurch gekennzeichnet, daß** die anorganischen Basen in Mengen von 1,1-2,0 vol pro Mol Carbazol der allgemeinen Formel II eingesetzt werden.

9. Verfahren nach einem oder mehreren der Ansprüche 1 bis 8, **dadurch gekennzeichnet, daß** als inerte Lösungsmittel Toluol, o-Xylol, m-Xylol, p-Xylol, Mesitylen, 2-Chlortoluol, 3-Chlortoluol, 4-Chlortoluol, Monochlorbenzol, 1,2-Dichlorbenzol, 1,3-Dichlorbenzol, 1,4-Dichlorbenzol, N-Ethylcarbazol oder deren Gemische einsetzt werden.

10. Verfahren nach einem oder mehreren der Ansprüche 1 bis 9, **dadurch gekennzeichnet, daß** in den Katalysatoren der allgemeinen Formel IV n eine ganze Zahl von 2 bis 6, bedeutet und daß R², R³, R⁴ und R⁵ bevorzugt für Wasserstoff, Methyl oder Ethyl, stehen.

11. Verfahren nach Anspruch 10, **dadurch gekennzeichnet, daß** inden Katalysatoren der allgemeinen Formel IV n die Zahl 3 bedeutet.

12. Verfahren nach Anspruch 11, **dadurch gekennzeichnet, daß** R², R³, R⁴ und R⁵ für Methyl stehen.

13. Verfahren nach einem oder mehreren der Ansprüche 1 bis 12, **dadurch gekennzeichnet, daß** als Katalysatoren der allgemeinen Formel IV Ethylendiamin, 2-Ethylamino-ethylamin, 2-Diethylamino-ethylamin, 3-Dimethylaminopropylamin, 3-Diethylamino-propylamin, N,N,N',N'-Tetramethyl-1,3-propandiamin, N,N-Diethyl-N',N'-dimethyl-1,3-propandiamin, N,N,N',N'-Tetramethyl-1,4-butandiamin, 1-Diethylamino-4-aminopentan, N,N,N',N'-Tetramethyl-1,6-hexandiamin, N,N-Dimethyldipropylentriamin, Bis-(3-dimethyl-aminopropyl)-amin, N,N,N',N",N"-Pentamethyldiethylentriamin oder N,N,N',N",N"-Pentamethyldipropylen-triamin eingesetzt werden

14. Verfahren nach einem oder mehreren der Ansprüche 1 bis 13 **dadurch gekennzeichnet, daß** die Katalysatoren der allgemeinen Formel IV in Mengen von 0,001 bis 0,05 Mol, pro Mol eingesetztes Carbazol, eingesetzt werden.

15. Verfahren nach Anspruch 14, **dadurch gekennzeichnet, daß** die Katalysatoren der allgemeinen Formel IV in Mengen von 0,005 bis 0,02 Mol pro Mol eingesetztes Carbazol eingesetzt werden.

## Claims

1. A process for preparing N-alkylcarbazoles of the formula I 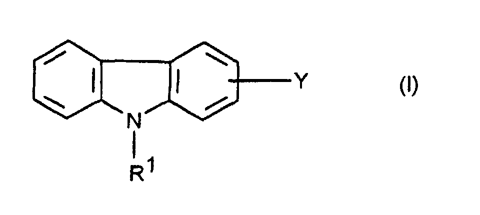 where
R¹ is (C₁-C₆)-alkyl and
Y is hydrogen, (C₁-C₆)-alkyl, (C₁-C₆)-alkoxy, nitro or halogen,
by reacting a carbazole of the formula II 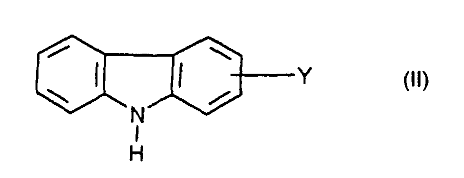 with an alkyl halide of the formula III
R¹-X III
where X is a halogen atom,
in an inert solvent in the presence of an inorganic base and a catalyst of the formula IV
R² R³ N-(CH₂)ₙ-NR⁴R⁵ IV
where
n is an integer from 2 to 8,
R², R³ and R⁴ are, independently of one another, hydrogen or (C₁-C₄)-alkyl and
R⁵ is hydrogen, (C₁-C₄)-alkyl.

2. The process as claimed in claim 1, wherein, in the compound of the formula I, R¹ is methyl or ethyl and Y is hydrogen.

3. The process as claimed in claim 1 and/or 2, wherein the alkyl halide of the formula III which is used is methyl chloride, methyl bromide, methyl iodide, ethyl chloride, ethyl bromide, n-propyl chloride, n-propyl bromide, iso-butyl chloride, iso-butyl bromide, n-pentyl chloride or n-hexyl chloride.

4. The process as claimed in one or more of claims 1 to 3, wherein the alkyl halides of the formula III are used in amounts of from 1.0 to 1.8 mol per mole of carbazole of the formula II.

5. The process as claimed in claim 4, wherein alkyl halides of the formula III are used in amounts of from 1.1 to 1.5 mol per mole of carbazole of the formula II.

6. The process as claimed in one or more of claims 1 to 5, wherein the inorganic bases used are alkali metal alkoxides and hydroxides, oxides, carbonates and also tertiary phosphates of the alkali metals and alkaline earth metals or mixtures thereof.

7. The process as claimed in one or more of claims 1 to 6, wherein the inorganic bases are used in amounts of from 0.9 to 3.0 eq per mole of carbazole of the formula II.

8. The process as claimed in claim 7, wherein the inorganic bases are used in amounts of from 1.1 to 2.0 eq per mole of carbazole of the formula II.

9. The process as claimed in one or more of claims 1 to 8, wherein the inert solvent used is toluene, oxylene, m-xylene, p-xylene, mesitylene, 2-chlorotoluene, 3-chlorotoluene, 4-chlorotoluene, monochlorobenzene, 1,2-dichlorobenzene, 1,3-dichlorobenzene, 1,4-dichlorobenzene, N-ethylcarbazole or a mixture thereof.

10. The process as claimed in one or more of claims 1 to 9, wherein, in the catalysts of the formula IV, n is an integer from 2 to 6, and R², R³, R⁴ and R⁵ are preferably hydrogen, methyl or ethyl.

11. The process as claimed in claim 10, wherein, in the catalysts of the formula IV, n is 3.

12. The process as claimed in claim 11, wherein R², R³, R⁴ and R⁵ are methyl.

13. The process as claimed in one or more of claims 1 to 12, wherein the catalyst of the formula IV which is used is ethylenediamine, 2-ethylaminoethylamine, 2-diethylaminoethylamine, 3-dimethylaminopropylamine, 3-diethylaminopropylamine, N,N,N',N'-tetramethylpropane-1,3-diamine, N,N-diethyl-N',N'-dimethylpropane-1,3-diamine, N,N,N',N'-tetramethylbutane-1,4-diamine, 1-diethylamino-4-aminopentane, N,N,N',N'-tetramethylhexane-1,6-diamine, N,N-dimethyldipropylenetriamine, bis(3-dimethylaminopropyl)amine, N,N,N',N'',N''-pentamethyldiethylenetriamine or N,N,N',N'',N''-pentamethyldipropylenetriamine.

14. The process as claimed in one or more of claims 1 to 13, wherein the catalysts of the formula IV are used in amounts of from 0.001 to 0.05 mol per mole of carbazole used.

15. The process as claimed in claim 14, wherein the catalysts of the formula IV are used in amounts of from 0.005 to 0.02 mol per mole of carbazole used.

## Revendications

1. Procédé pour la préparation de N-alkylcarbazoles de formule générale I 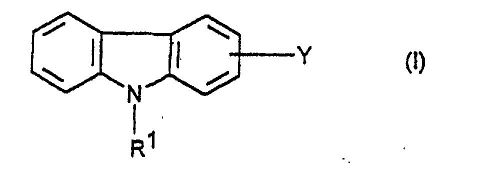 dans laquelle
R¹ représente un groupe alkyle en C₁ à C₆ et
Y représente un atome d'hydrogène, un groupe alkyle en C₁ à C₆, alcoxy en C₁ à C₆, nitro ou un atome d'halogène,
par réaction d'un carbazole de formule générale II 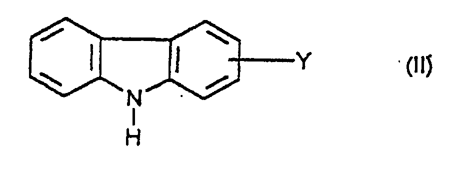 avec un halogénure d'alkyle de formule générale III
R¹-X III
dans laquelle X représente un atome d'halogène,
dans un solvant inerte en présence d'une base inorganique et d'un catalyseur de formule générale IV
R²R³N-(CH₂)ₙ-NR⁴R⁵ IV
dans laquelle
n représente un nombre entier compris entre 2 et 8,
R², R³ et R⁴ représentent indépendamment les uns des autres un atome d'hydrogène ou un groupe alkyle en C₁ à C₄ et
R⁵ représente un atome d'hydrogène, un groupe alkyle en C₁ à C₄.

2. Procédé selon la revendication 1, **caractérisé en ce que** dans le composé de formule générale I R¹ représente le méthyle ou l'éthyle et Y un atome d'hydrogène.

3. Procédé selon la revendication 1 et/ou 2, **caractérisé en ce qu'**on utilise comme halogénure d'alkyle de formule générale III le chlorure de méthyle, le bromure de méthyle, l'iodure de méthyle, le chlorure d'éthyle, le bromure d'éthyle, le chlorure de n-propyle, le bromure de n-propyle, le chlorure d'isobutyle, le bromure d'isobutyle, le chlorure de n-pentyle ou le chlorure de n-hexyle.

4. Procédé selon une ou plusieurs des revendications 1 à 3, **caractérisé en ce que** les halogénures d'alkyle de formule générale III sont mis en oeuvre en quantités de 1,0 à 1,8 mol, par mol de carbazole de formule générale II.

5. Procédé selon la revendication 4, **caractérisé en ce que** les halogénures d'alkyle de formule générale III sont mis en oeuvre en quantités de 1,1 à 1,5 mol, par mol de carbazole de formule générale II.

6. Procédé selon une ou plusieurs des revendications 1 à 5, **caractérisé en ce qu'**on utilise comme bases inorganiques des alcoolates de métaux alcalins et des hydroxydes, oxydes, carbonates et phosphates tertiaires des métaux alcalins et des métaux alcalino-terreux ou leurs mélanges.

7. Procédé selon une ou plusieurs des revendications 1 à 6, **caractérisé en ce qu'**on utilise les bases inorganiques en quantités comprises entre 0,9 et 3,0 val, par mol de carbazole de formule générale II.

8. Procédé selon la revendication 7, **caractérisé en ce qu'**on utilise les bases inorganiques en quantités comprises entre 1,1 et 2,0 val, par mol de carbazole de formule générale II.

9. Procédé selon une ou plusieurs des revendications 1 à 8, **caractérisé en ce qu'**on utilise comme solvants inertes le toluène, l'o-xylène, le m-xylène, le p-xylène, le mésitylène, le 2-chlorotoluène, le 3-chlorotoluène, le 4-chlorotoluène, le monochlorobenzène, le 1,2-dichlorobenzène, le 1,3-dichlorobenzène, le 1,4-dichlorobenzène, le N-éthylcarbazole ou leurs mélanges.

10. Procédé selon une ou plusieurs des revendications 1 à 9, **caractérisé en ce que** dans les catalyseurs de formule générale IV n représente un nombre entier compris entre 2 et 6, et **en ce que** R², R³, R⁴ et R⁵ représentent de préférence un atome d'hydrogène, un groupe méthyle ou éthyle.

11. Procédé selon la revendication 10, **caractérisé en ce que** dans les catalyseurs de formule générale IV, n représente le nombre 3.

12. Procédé selon la revendication 11, **caractérisé en ce que** R², R³, R⁴ et R⁵ représentent un groupe méthyle.

13. Procédé selon une ou plusieurs des revendications 1 à 12, **caractérisé en ce qu'**on utilise comme catalyseurs de formule générale IV l'éthylènediamine, la 2-éthylamino-éthylamine, la 2-diéthylamino-éthylamine, la 3-diméthylamino-propylamine, la 3-diéthylaminopropylamine, la N,N,N',N'-tétraméthyl-1,3-propanediamine, la N,N-diéthyl-N',N'-diméthyl-1,3-propanediamine, la N,N,N',N'-tétraméthyl-1,4-butanediamine, le 1-diéthylamino-4-aminopentane, la N,N,N',N'-tétraméthyl-1,6-hexanediamine, la N,N-diméthyldipropylènetriamine, la bis-(3-diméthylaminopropyl)-amine, la N,N,N',N'',N''-pentaméthyldiéthylènetriamine ou la N,N,N',N'',N''-pentaméthyldipropylènetriamine.

14. Procédé selon une ou plusieurs des revendications 1 à 13, **caractérisé en ce que** les catalyseurs de formule générale IV sont utilisés en quantités de 0,001 à 0,05 mol par mol de carbazole mis en oeuvre.

15. Procédé selon la revendication 14, **caractérisé en ce que** les catalyseurs de formule générale IV sont utilisés en quantités de 0,005 à 0,02 mol par mol de carbazole mis en oeuvre.
